# EUROPEAN PATENT APPLICATION

(11) **EP 1 138 779 A2**
(43) Date of publication of application: **04.10.2001**
(21) Application number: 01302365.0
(22) Date of filing: 14.03.2001
(51) Int. Cl.: C12Q 1/26, G01N 33/94

(54) **Methods of rapid screening of cytochrome CYP2C19 status using mephenytoin**

(30) Priority: 30.03.2000 US 193099 P
(71) Applicant: Pfizer Products Inc., Groton, Connecticut 06340 (US)
(72) Inventor: Friedman, Hylar Lewis, Groton, Connecticut 06340 (US); Shah, Ajitkumar Kantilal, Groton, Connecticut 06340 (US)
(74) Representative: Hayles, James Richard

(57) **Abstract**

Mammalian subjects may be phenotyped as poor or extensive metabolizers with respect to CYP2C19 by measuring the ratio of R and S mephenytoin or S-mephenytoin to S-4-hydroxymephenytoin produced from racemic mephenytoin in urine, saliva or plasma samples.

## Description

### FIELD OF THE INVENTION

This invention is directed toward methods of rapidly screening subjects for poor and extensive CYP4502C19 (CYP2C19) activity.

### BACKGROUND OF THE INVENTION

There are wide individual variations in drug efficacy and toxicity. To a great extent, these differences are the result of differences in the metabolism, distribution and elimination of the therapeutic agents. While physiological factors affecting distribution (body mass, albumin levels, etc.) and elimination (kidney function) are relatively easy to measure, the assessment of metabolic capacity is not a routine procedure. Part of this is due to the fact that metabolizing enzymes exhibit a large degree of individual variability in their levels of expression. Some polymorphic metabolic abnormalities are understood at the DNA level (genotype) but for the majority, the underlying mutations have not been identified. In such cases, metabolic phenotyping remains the only way to assess metabolic capacity through the identification of specific metabolite patterns produced by 'probe drugs'.

In probe drug phenotyping, urine or blood samples are collected before and after administration of the probe metabolized by specific enzyme(s) and responsible for the production of a particular metabolite, then metabolite to parent ratios can be used to derive phenotypes. Individuals who are deficient in their ability to metabolize the probe drug are called 'slow' or 'poor' metabolizers (pm). Those who have a normal or greater than average metabolic activity are called 'fast' or 'extensive' metabolizers (em).

While the genotype prescribes the native levels of enzymatic activity, changes in the relative levels and activities of metabolizing enzymes can be produced by drug interactions and/or clinical conditions such as disease progression or malnutrition. Thus a relatively healthy patient will not necessarily have the same phenotype to a drug when that person's health has degenerated. This phenomenon has been suggested in AIDS patients for the activity of the enzyme N-acetyltransferase 2 (NAT2). In this case, the NAT2 phenotype appears to change in AIDS patients from "fast" to "slow" while the genotype remains constant.

The key barrier in the routine incorporation of metabolic activity assessments in clinical treatment is the lack of rapid, inexpensive and 'user friendly' methods for these measurements. (Ducharme, J. of Chromat. B, 678 (1996) 113-28)

M. Eickelbaum and B. Evert reviewed 5- mephenytoin polymorphism related to cytochrome P450 2C19 (CYP2C19)³.

This polymorphism was discovered by Küpfer and colleagues¹. They observed a subject who, very slowly, cleared the antiepileptic drug mephenytoin. The major metabolite is 4-hydroxy/mephenytoin, which is formed mainly from the S-enantiomer, whereas the R-enantiomer is much more slowly cleared from the body by N-demethylation. Poor metabolizers (pm) form only trace amounts of the hydroxylated metabolite of S-enantiomer and, as a consequence, have a much lower clearance. Phenotype assignment is now based on the ratio of S to R enantiomer, PM having a ratio of approximately 1 and EM below this value. The defect is inherited as an autosomal trait. The frequency of PM shows substantial racial differences with 2 - 3% occurring in Caucasians and up to 23% in Oriental populations². It was not until recently that CYP2C19 was identified as the enzyme that catalysed the metabolism of mephenytoin.

### References

1. Küpfer A, Desmond P, Schenker S, Branch RA. Family study of a genetically determined deficiency of mephenytoin hydroxylation in man. Pharmacogenetics 1994; 21: 173.
2. Bertilsson L. Geographical/interracial differences in polymorphic drug oxidation. Current state of the knowledge of cytochromes P450(CYP)2D6 and 2C19. Clin. Pharmacokin. 1995; 29:192-209.
3. Clinical and Experimental Pharmacology and Physiology (1996) 23, 983-5.

### SUMMARY OF THE INVENTION

Oxidative metabolism involving the cytochrome P450 2C19 CYP2C19 is genetically determined. This results in some individuals being phenotyped as "poor" and others as "extensive" metabolizers. Typically, the urinary molar concentration ratio of a probe drug to metabolite over an interval of 8-10 hours is determined to assign phenotype. We evaluated methods to determine CYP2C19 phenotype which may be useful to rapidly screen subjects in clinical trials using racemic mephenytoin.

In a small group of subjects our results showed that at least a 4 hour urine collection, 1 hour plasma, or 2 hour saliva sample post-dose accurately predicted CYP2C19 phenotype.

This invention provides method for phenotyping a subject as a "poor" or "extensive" metabolizer which comprises:
a) administering a dose of racemic mephenytoin to said subject;
b) waiting for a period of time;
c) obtaining a sample of urine, plasma or saliva from said subject;
d) measuring the concentrations of racemic mephenytoin and R and S mephenytoin enantiomers in said sample.

In another aspect this invention provides a method wherein said sample is a urine sample.

In another aspect this invention provides a method wherein said sample is a saliva sample.

In another aspect this invention provides a method wherein said sample is a plasma sample.

In another aspect this invention provides a method wherein said period of time is one to four hours.

In another aspect this invention provides a method wherein said dose of racemic mephenytoin is 100 mg.

### DETAILED DESCRIPTION OF THE INVENTION

The liver contains enzymes that convert various drug substances to products, called metabolites, which can be more easily eliminated from the body, usually in the urine or feces. This conversion process, which is also known as chemical metabolism or chemical biotransformation, frequently determines the duration of action of drugs or the intensity of the drug action, which is why drugs must typically be taken several times each day to treat diseases and produce other desirable pharmacological effects.

The many xenobiotic-metabolizing enzyme systems of the liver include cytochrome P450, carboxylesterases, UDP-glucuronosyltransferases, sulfotransferases, glutathione S-transferases and many others. Each of these enzyme systems is comprised of numerous individual enzymes, each of which is capable of metabolizing a wide variety of pharmaceuticals and other chemical compositions. For example, the cytochrome P450 system in the human liver is comprised of at least ten individual P450 enzymes. Of these various enzyme systems, the P450 enzymes play the most important role in determining the rate of elimination of drugs.

Metabolism by cytochrome P450 often represents the rate-limiting step in pharmaceutical elimination. Consequently, factors that lessen the activity of P450 enzymes usually prolong the effects of pharmaceuticals, whereas factors that increase cytochrome P450 activity have the toxic effect.

Changes in drug metabolism may have undesirable or toxic consequences. For example, impaired metabolism of a drugs by factors that decrease cytochrome P450 activity have the toxic effect.

Conversely, the accelerated metabolism of a drug due to increased concentrations of cytochrome P450 can also lead to a lessening of therapeutic effect.

Information from phenotyping can also be used to explain or predict adverse drug reactions that result from variances in the activity of various P450 enzymes. During phenotyping, the quantity and/or kind of metabolites produced when a drug reacts with an enzyme are used to identify the existence of that enzyme in an individual. The therapeutic or toxic effects of certain drugs can be exaggerated or compounded in a significant percentage of the population-at-large due to a genetic deficiency in a CYP P450 enzyme.

Poor metabolizers often experience exaggerated responses to drugs at dosages that are well tolerated by normal subjects who have a better capability to metabolize the same drugs. If a drug is metabolized by cytochrome CYP2C19, the drug likely will have an exaggerated or toxic effect in individuals lacking CYP2C19.

It is highly desirable to provide a method for identifying "poor" and "extensive" metabolizers of drugs. The present invention provides a simple and rapid method for identifying individuals who are "poor" and "extensive" metabolizers based on CYP2C19 activity.

In this method, a fasting subject is dosed with an effective amount of mephenytoin and the ratios of S to R mephenytoin enantiomers and S-mephenytoin to S-4-hydroxymephenytoin in saliva, plasma or urine are determined by LC/MS/MS. A urinary S to R enantiomers ratio of > 0.9 classifies a subject as a "poor" metabolizer and a ratio of < 0.7 classifies a subject as an "extensive" metabolizer. Using urinary hydroxylation ratio (S-mephenytoin to S-4-hydroxymephenytoin) from 0.6 to 20 defines "extensive" metabolizers while in "poor" metabolizers, it ranges from 30 to 2500.

### EXAMPLE 1

Oxidative metabolism involving the cytochrome P450 2C19 (CYP2C19) is genetically determined. This results in some individuals being phenotyped as poor and others as extensive metabolizers. Typically, the plasma molar concentration ratio of a probe drug to metabolite is determined to assign phenotype. A total of 32 healthy male and female subjects (age 30 +/- year and weight 76 +/- 9.2 kg, including 24 extensive and 8 poor metabolizers based on genotyping) were enrolled. After an ovemight fast subjects received a single 100mg dose of racemic mephenytoin. Serial hourly plasma and saliva samples, and urine over every 2 hour interval were obtained during the 8 hour study period. Samples were assayed for R and S enantiomers, and R and S-4-hydroxymephenytoin by LC/MS/MS method. The molar ratios S to R mephenytoin enantiomers and S-mephenytoin to S-4-hydroxymephenytoin were determined. Using S-mephenytoin to S-4-hydroxymephenytoin ratio, a plasma sample at any time within eight hours while S to R enantiomers ratio at anytime between 3 to 8 hours post mephenytoin dose discriminated between extensive and poor metabolizers. Saliva results for S to R enantiomers at any time between 2 to 8 hours post dose discriminated between extensive and poor metabolizer. Urine samples over intervals 2-4, 4-6, and 6-8 hours using S-mephenytoin to S-4-hydroxymephenytoin ratio discriminated between EM and PM. The results of this small study provide alternative methods to use either plasma, saliva, or urine samples to determine CYP2C19 phenotypes.

## Claims

1. A method for phenotyping a mammalian subject as a poor or extensive metabolizer with respect to CYP2C19 which comprises:
a) administering a dose of racemic mephenytoin to said subject;
b) waiting for a period of time;
c) obtaining a sample of urine, plasma or saliva from said subject;
d) measuring the concentrations of S and R mephenytoin enantiomers.

2. A method of claim 1 wherein said sample is a saliva sample.

3. A method of claim 1 wherein said is a plasma sample.

4. A method of claim 1 wherein said sample is a urine sample.

5. A method of claim 2 wherein said period of time is two hours.

6. A method of claim 1 wherein said dose is 100 mg of racemic mephenytoin.

7. A method of claim 3 wherein said period of time is anytime within 8 hours.
